# EUROPEAN PATENT APPLICATION

(11) **EP 1 944 557 A2**
(43) Date of publication of application: **16.07.2008**
(21) Application number: 08000411.2
(22) Date of filing: 10.01.2008
(51) Int. Cl.: F24F 1/00, F24F 3/16

(54) **Air filtering apparatus**

(30) Priority: 12.01.2007 JP 2007004043
(71) Applicant: Sanyo Electric Co., Ltd., Moriguchi-shi, Osaka (JP)
(72) Inventor: Dobashi, Mitsuhiro, Kumagaya-shi Saitama 360-0033 (JP); Fukushima, Toshio, Ota-shi Gunma 373-0038 (JP); Kobayashi, Hiroyuki, Ora-gun Gunma 370-0533 (JP); Yamamoto, Tetsuya, Ota-shi Gunma 373-0806 (JP); Nishihara, Takuro, Ota-shi Gunma 373-0817 (JP); Okunaka, Toshinori, Ashikaga-shi Tochigi 326-0016 (JP); Arakawa, Toru, Tatebayashi-shi Gunma 372-0066 (JP); Usui, Hiroaki, Ora-gun Gunma 370-0523 (JP)
(74) Representative: Glawe, Delfs, Moll

(57) **Abstract**

An air filtering apparatus that is equipped with an air blower (21) and a gas-liquid contact member (31) in a housing (10) and in which electrolytic water is brought into contact with indoor air sucked by the air blower to filter the air, and then the filtered air is blown out to an indoor space further comprises a water supply tank (41)for stocking water, a water receiving tray (44) for temporarily stocking water flowing out from the water supply tank (41), a circulating pump (42) for pumping up water stocked in the water receiving tray, an electrolytic water generator (43) for generating electrolytic water from the water pumped by the circulating pump (42), and an electrolytic water circulating passage for circulating electrolytic water through the gas-liquid contact member and the water receiving tray in this order, wherein at least one ion exchange resin pack (100) for adjusting the hardness of water is provided in the water supply tank is located out of the circulating passage of the electrolytic water.

## Description

### BACKGROUND OF THE INVENTION

### 1. Field of the Invention

The present invention relates to an air filtering apparatus that can remove microorganisms floating in the air (bacteria, virus, fungus, etc. (hereinafter merely referred to as "virus, etc.").

### 2. Description of the Related Art

For the purpose of remove virus, etc. floating in the air, there has been proposed an air filtering apparatus in which water such as tap water or the like is electrolyzed to generate electrolytic water containing active oxygen species such as hypochlorous acid or the like, the electrolytic water is supplied to a humidifying element (a filtering member, a gas-liquid contact member or the like) formed of non-woven cloth or the like, and air containing virus, etc. is brought into contact with the electrolytic water supplied to the humidifying element to inactivate microorganisms (virus, etc.) floating in the air, thereby filtering the air (for example, see JP-A-2002-181358). In this type of air filtering apparatus, a pair of electrodes are provided, and current is made to flow between the electrodes to thereby electrolyze tap water or the like and thus generate electrolytic water.

When tap water or the like is electrolyzed to generate electrolytic water in the above-described air filtering apparatus, scale is liable to occur due to water quality such as hardness or the like of tap water or the like.

The water quality of tap water is different among countries in the world or among districts even in each country. For example, when the hardness is equal to 100ppm or the like, scale occurs in a short time, and thus the electrodes for generating electrolytic water, pipes for supplying electrolytic water to the gas-liquid contact member, etc. are liable to clog with the scale. In order to solve this problem, the maintenance time of water exchange must be set to a short time, for example, about 40 hours. There is generally known ion exchange resin in which the hardness of water is adjusted so that water having high hardness is softened. However, the ion exchange resin can be regenerated, but the amount of water which can be softened by one regenerating operation is limited.

Furthermore, the concentration of chlorine ions contained in tap water is different among respective countries in the world or among districts in each country, and there is a case where electrolytic water containing active oxygen species of a predetermined concentration required for air filtering cannot be generated even when power is supplied between the electrodes with current of a predetermined current value for a predetermined current supply time. The current supply state of the electrodes is subjected to default setting on the basis of the water quality of standard tap water. However, when an air filtering apparatus is set in a predetermined district, the current supply state of the electrodes must be changed in accordance with the water quality of tap water in that district. However, after the air filtering apparatus is once set, variation of the water quality of tap water is rare, and thus there is little opportunity of changing the setting concerning the current supply state. Furthermore, when the setting concerning the current supply state is carelessly changed, the concentration of active oxygen species contained in electrolytic water is varied, and a desired air filtering effect cannot be obtained.

### SUMMARY OF THE INVENTION

Therefore, the present invention has been implemented to solve the above problem, and has an object to provide an air filtering apparatus that can suppress occurrence of scale in an electrolysis process, so that an interval time required for water exchange can be set to a long value.

In order to attain the above obj ect, according to the present invention, an air filtering apparatus that is equipped with an air blower (21) and a gas-liquid contact member (31) in a housing (10) and in which electrolytic water is brought into contact with indoor air sucked by the air blower to filter the air, and then the filtered air is blown out to an indoor space is characterized by further comprising: a water supply tank (41)for stocking water; a water receiving tray (44) for temporarily stocking water flowing out from the water supply tank (41) ; a circulating pump (42) for pumping up water stocked in the water receiving tray; an electrolytic water generator (43) for generating electrolytic water from the water pumped by the circulating pump (42); and an electrolytic water circulating passage for circulating electrolytic water through the gas-liquid contact member and the water receiving tray in this order, wherein at least one ion exchange resin pack (100) for adjusting the hardness of water is provided in the water supply tank is located out of the circulating passage of the electrolytic water.

In this invention, the ion exchange resin pack for softening water such as tap water or the like is provided in the water supply tank located out of the electrolytic water circulating passage. Therefore, the circulating electrolytic water is not brought into contact with the ion exchange resin pack. That is, the ion exchange resin pack is used only to soften water in the water supply tank, and it does not come into contact with the circulating electrolytic water.

The circulating electrolytic water has been already softened, and it is unnecessary to bring the electrolytic water into contact with the ion exchange resin. Therefore, in this construction, the interval time required to generate the ion exchange resin can be set to a longer time because the ion exchanger resin is not brought into contact with the circulating electrolytic water.

Furthermore, if the ion exchange resin pack is set to such a state that a predetermined amount of ion exchange resin is filled in a pack, water can be simply softened by merely changing the number of ion exchange resin packs to be input into the water supply tank.

In this case, the electrolytic water generator has at least a pair of electrodes and a controller for controlling the current supply state between these electrodes, and the controller may be provided with the operating unit for changing the current supply state between the electrodes.

In this construction, even when the current supply state between the electrodes is set to a default value on the basis of the water quality of standard tap water in advance and shipped from a factory, the current supply state between the electrodes can be simply changed by operating the operating unit.

Furthermore, the operating unit may be disposed so as to be exposed to the outside from the hole formed in the electrical component box in which the controller is accommodated.

The operating unit is exposed to the outside of the electrical component box from the hole formed in the electrical component box in which the controller is mounted, and thus the current supply state in the electrolysis process can be changed without opening the electrical component box. Furthermore, the electrical component box is disposed in the housing, and thus the current supply state between the electrodes can be prevented from being carelessly changed by a user or the like.

In the air filtering apparatus having the above construction, it is preferable that the operating unit stepwise change the current supply state between the electrodes to one of preset current supply states of plural stages by rotating the operating unit.

In this construction, the user or the like can simply change the current supply state to any one of the preset plural current supply states of plural stages by the rotating the rotating operational tab.

Furthermore, it is preferable that the housing is provided with the freely-detachable outer packaging panel, and the electrical component box is disposed in the housing so that the operating unit is exposed to the outside.

In this construction, the electrical component box is disposed in the housing so that the operating unit is exposed to the outside when the outer packaging panel is detached. Therefore, the current supply state between the electrodes can be easily changed by detaching the outer packaging panel. Furthermore, by attaching the outer packaging panel, the operating unit can be prevented from being exposed to the outside of the housing and also the current supply state between the electrodes can be prevented from being carelessly changed by the user or the like.

It is preferable that the electrical component box is provided with a peaked portion projecting to the outside of the electrical component box, and the operating unit is provided below the peaked portion.

In this construction, the electrical component box is provided with the peaked portion projecting to the outside of the electrical component box, and the operating unit is provided below the peaked portion. The operating unit is disposed so as to be exposed to the outside of the electrical component box from the hole formed in the electrical component box, and thus water can be prevented from invading into the hole by the peaked portion. The operating unit may change a power amount to be supplied to the electrodes, change a current supply time to supply current between the electrodes, or change a current value flowing between the electrodes.

According to the present invention, the ion exchange resin pack for softening tap water is provided in the water supply tank located out of the circulating passage of electrolytic water. Therefore, the ion exchange resin pack is not brought into contact with the circulating electrolytic water. Accordingly, the interval time required to regenerate the ion exchange resin can be set to a longer time because the ion exchange resin is not brought into contact with the circulating electrolytic water.

Furthermore, the ion exchange resin pack 100 is constructed by packing a predetermined amount of ion exchange resin, and water in the water supply tank can be simply softened by merely changing the number of ion exchange resin packs to be input into the water supply tank in accordance with the hardness of water.

### BRIEF DESCRIPTION OF THE DRAWINGS

Fig. 1 is a perspective view showing an example of the external view of an air filtering apparatus according to the present invention;
Fig. 2 is a perspective view showing an example of the internal view of the air filtering apparatus according to the present invention;
Fig. 3 is a side cross-sectional view showing the air filtering apparatus according to the present invention;
Fig. 4 is a diagram showing a circulating path of electrolytic water in the air filtering apparatus according to the present invention;
Fig. 5 is a diagram showing water quality adjustment which is carried out in accordance with hardness and chlorine ion concentration;
Figs. 6A is a perspective view showing an example of the external view of an electrical equipment box equipped in the housing of the air filtering apparatus of the present invention, and Fig. 6B is a perspective view showing an example of the internal view of the electrical equipment box;
Fig. 7 is a front view showing an arrangement indicated by a broken line in the housing of the electrical equipment box according to the present invention;
Fig. 8 is a diagram showing the construction of operating means according to the present invention.

### DETAILED DESCRIPTION OF THE PREFERRED EMBODIMENTS

Preferred embodiments according to the present invention will be described hereunder with reference to the accompanying drawings.

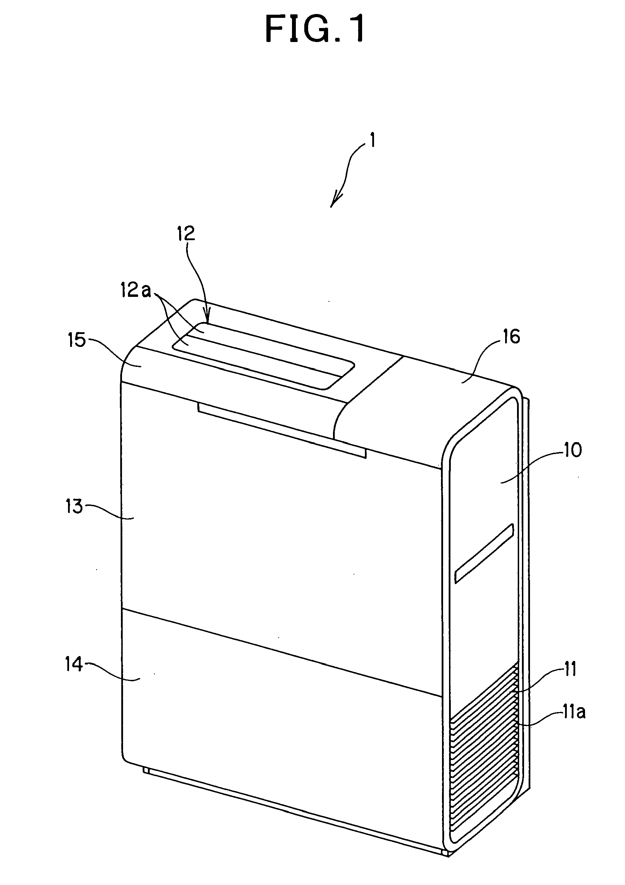

Fig. 1 is a perspective view showing the outlook of an air filtering apparatus 1 according to an embodiment. In the air filtering apparatus 1, electrolytic water containing predetermined active oxygen species is generated by using tap water or the like, indoor air sucked into the air filtering apparatus 1 is filtered by using the electrolytic water and clean air after the air filtering is blown into the room.

As shown in Fig. 1, the air filtering apparatus 1 is equipped with a box-shaped housing 10 formed in an vertically long rectangular parallelepiped shape, and it is designed as an on-floor mount type. Air suction ports 11 for sucking air are formed in both the side surfaces of the housing 10, and an air blow-out port 12 for blowing out air is formed in the top surface of the housing 10. Each air suction port 11 is provided with an air suction grille 11a having a plurality of elongated vanes arranged in parallel in the horizontal direction, and an automatic louver 12a for changing the air blowing direction is provided to the air blow-out port 12.

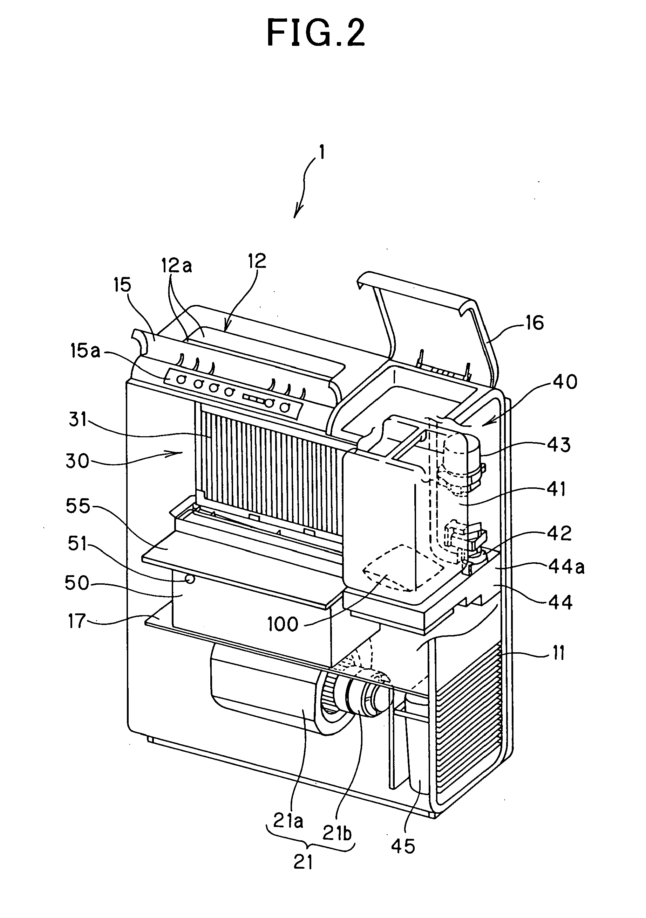

An upper front panel 13 (outer package panel) and a lower front panel 14 are provided to the front surface (face) side of the housing 10, and these panels 13 and 14 are designed to be freely opened and closed. Furthermore, an elongated operation panel 15a (see Fig. 2) which is formed in parallel to the air blow-out port 12 in front of the air blow-out port 12 is provided to the top surface of the lower front panel, and a lid 15 which is freely opened and closed is provided to the operation panel 15a. Furthermore, an opening/closing lid 16 through which a water supply tank 41 described later (see Fig. 2) is put in and out is provided to one side of the top surface (the right side in front view) of the housing 10.

Next, the internal construction of the air filtering apparatus 1 will be described with reference to Figs. 2 to 4.

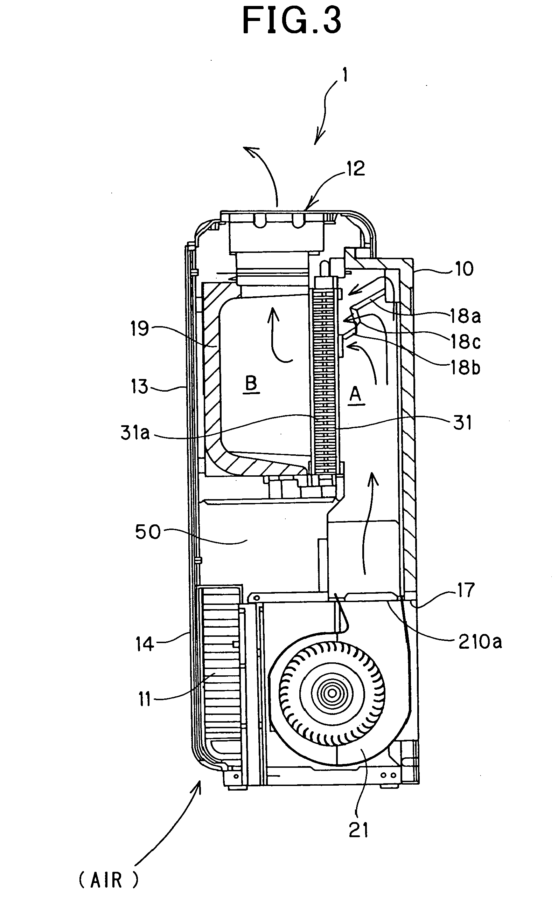

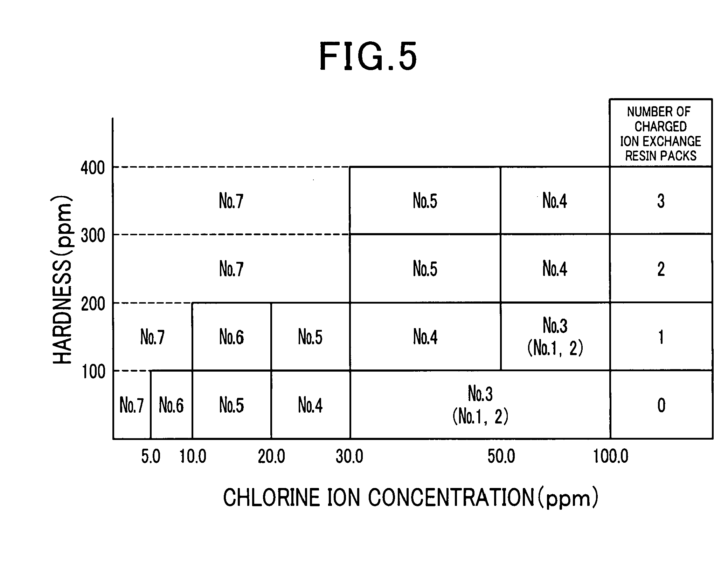

Fig. 2 is a perspective view showing the main construction of the inside of the air filtering apparatus 1. Fig. 3 is a side cross-sectional view. As shown in Figs. 2 and 3, the air filtering apparatus 1 is equipped with an air blower 21 for forming an air flowing path extending from the air suction ports 11 to the air blow-out port 12 in the housing 10, an air filtering unit 30 having a gas-liquid contact member 31 for bringing electrolytic water into contact with air supplied through the air flowing path, a water supply tank 41 as a constituent element of an electrolytic water circulating and supplying unit 40 for circulatinglysupplyelectrolyticwatertothegas-liquidcontact member 31, a circulating pump 42, an electrolytic bath (electrolytic water generator) 43 and a water receiving tray 44. Furthermore, a controller 52 (see Fig. 5) for controlling respective parts in the air filtering apparatus 1 and an electrical equipment box 50 in which various other kinds of electrical components are accommodated are disposed in the housing 10. An example of the arrangement of these units will be described hereunder.

As shown in Figs. 2 and 3, the inside of the housing 10 is vertically divided into upper and lower parts by a partition plate 17. The air blower 21, a drain tank 45 for discharging electrolytic water stocked in the water receiving tray 44, etc., and other units are disposed in the lower part divided by the partition plate 17.

As shown in Figs. 2 and 3, the air blower 21 has an air blowing fan 21a, and a driving motor 21b for driving the air blowing fan 21a. As shown in Fig. 1, the air blowing fan 21a is disposed at the left side of the housing 10 in front view, and an air flowing port 210a thereof is provided at the back side portion of the housing 10 with being placed face up (see Fig. 3). An opening is formed in the partition plate 17 so as to be located above the air flowing port 210a of the air blowing fan 21a. This opening intercommunicates with the space extending vertically at the back side of the housing (hereinafter referred to as "back side space A"). Accordingly, air sucked from the air suction ports 11 by the air flowing fan 21a directs from the lower front side of the housing 10 to the back side thereof, and flows upwardly in the back side space A of the housing 10.

As shown in Fig. 3, a front side space B is formed at the upper side of the partition plate 17 together with the back side space A, and the gas-liquid contact member 31 is erected so as to partition the back side space A and the front side space B.

The gas-liquid contact member 31 is a member for bringing electrolytic water into contact with air supplied through the air flowing path to the gas-liquid contact face 31a. In the gas-liquid contact member 31, air sucked into the housing 10 is brought into contact with electrolytic water containing predetermined active oxygen species, so that virus, etc. contained in the air is inactivated, thereby filtering the air.

The gas-liquid contact member 31 may be constructed by using a filter member (filter element) having a honeycomb structure (not shown). By designing the gas-liquid contact member 31 in the honeycomb structure, a large gas-liquid contact area can be secured, electrolytic water can be dropped, and also the structure which hardly clogs can be obtained. As the gas-liquid contact member 31 may be used a material having little reactivity to electrolytic water, that is, a material which is hardly deteriorated by electrolytic water, for example, polyolefin type resin (polyethylene resin, polypropylene resin or the like), PET (polyethylene terephthalate) resin, vinyl chloride resin, fluorinated resin) (PTFE, PFA, ETFE or the like), cellulose type material, ceramics type material or the like.

As shown in Fig. 3, two air guide plates 18a and 18b are provided at the upper portion of the back side space A so as to downwardly slope from the back side of the housing 10 to the front side thereof and also so as to be different positionally in the height direction, and the two air guide plates 18a and 18b are supported by a frame member 18c. Air which is blown upwardly in the back side space A abuts against the two air guide plates 18a and 18b, and it is passed through paths indicated by arrows of Fig. 3 and then supplied to the gas-liquid contact member 31. The air passed through the gas-liquid contact member 31 flows upwardly in the front side space B, and it is blown out from the air blow-out port 12 intercommunicating with the front side space B into the room. A housing 19 is provided between the gas-liquid contact member 31 and the upper front panel 13 to form the air flow path and prevent water splash of electrolytic water, etc. from the gas-liquid contact member 31, etc.

As shown in Fig. 2, in addition to the gas-liquid contact member 31, the electrical component box 50 is mounted above the partition plate 17, particularly at the left side above the partition plate 17 in front view. The water receiving tray 44 is disposed above the electrical component box 50, and the gas-liquid contact member 31 is disposed through the water receiving tray 44. The water supply tank 41 disposed below the opening/closing lid 16, the electrolytic bath 43 disposed behind the water supply tank 41, and a circulating pump 42 for supplying water in the water receiving tray 44 to the electrolytic bath 43 and the gas-liquid contact member 31 are disposed at the right side above the partition plate 17, thereby constructing the electrolytic water circulating and supplying unit 40.

The water supply tank 41 is a tank for stocking tap water or the like used to generate electrolytic water. A water supply port of the water supply tank 41 is disposed in a stock portion 44a of the water receiving tray 44 to supply a proper amount of water to the water receiving tray 44. However, the water to be supplied to generate electrolytic water is not limited to tap water, and well water, pure water, purified water or the like may be used in spite of tap water. The electrolytic bath 43 is designed so that electrolytic water is generated by electrolyzing water (containing electrolytic water generated in the electrolytic bath 43) as described later, and thus it is preferable that water containing a predetermined ion species such as chlorine ion or the like is stocked in the water supply tank 41. Therefore, when tap water having a low concentration of ion species, well water, pure water, purified water or the like is used, for example, it is preferable to add material containing a predetermined ion species such as salt or the like as electrolysis promoting agent for promoting electrolysis.

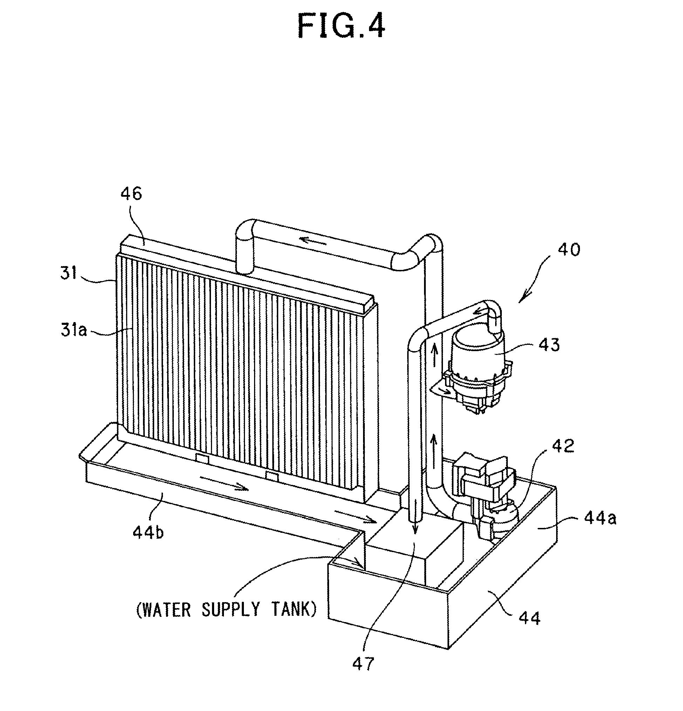

As shown in Fig. 4, a water spray box 46 which is one of the constituent elements of the electrolysis water circulating and supplying unit 40 is installed above the gas-liquid contact member 31 and the water receiving tray 44 is disposed below the gas-liquid contact member 31. The water receiving tray 44 is equipped with the stock portion 44a in which the water suction port of the water supply tank 41 is disposed, and a water receiving portion 44b which is disposed below the gas-liquid contact member 31 and receives electrolytic water discharged from the gas-liquid contact member 31. The circulation pump 42 is disposed in the stock portion 44a, and a filter member 47 for removing insoluble materials such as scale, etc. contained in the electrolytic water generated in the electrolytic bath 43 is disposed at the interconnection portion between the water receiving portion 44b and the stock portion 44a. The insoluble materials contained in the electrolytic water discharged from the gas-liquid contact member 31 are removed by the filter member 47. By disposing the filter member 47 as described above, insoluble materials such as scale, pollen, etc. are prevented from being contaminated into water pumped by the circulation pump 42.

In this embodiment, as indicated by arrows of Fig. 4, a part of water pumped by the circulating pump 42 is supplied to the gas-liquid contact member 31 through the water spray box 46, and the remaining water is supplied to the electrolytic bath 43. The electrolytic water generated in the electrolytic bath 43 is supplied to the stock portion 44a through the filter member 47, and the electrolytic water stocked in the stock portion 44a is dispersively supplied to the gas-liquid contact member 31 and the electrolytic bath 43 again by the circulating pupm 42. As described above, electrolysis is repetitively carried out by using electrolytic water in the electrolytic bath 43, whereby electrolytic water containing a high concentration of active oxygen species can be generated. Furthermore, by circulatingly using electrolytic water discharged from the gas-liquid contact member 31, only a small amount of water is supplied from the water supply tank 41 so as to make up a shortfall of water, so that water resource can be effectively used.

Furthermore, a drain hole (not shown) is formed in the bottom portion of the stock portion 44a, and it is connected to the drain tank 45 through a drain pipe (not shown). A drain valve (not shown) is provided to the drain pipe, and water in the water receiving tray 44 can be discharged to the drain tank 45 connected to the drain pipe by opening the drain valve.

Next, the electrolytic bath 43 will be described.

The electrolytic water contains at least a pair of electrodes (not shown). When a voltage is applied between the electrodes, water such as tap water or the like flowing into the electrolytic bath 43 is electrolyzed to generate electrolytic water containing active oxygen species. Here, the active oxygen species is oxygen having higher oxidizing activity than normal oxygen and relevant materials thereto, and contain not only so-called narrowly-defined active oxygen such as superoxide anion, singlet oxygen, hydroxyl radical and hydrogen peroxide, but also so-called broadly-defined active oxygen such as ozone, hypochlorous acid, hypohalous acid, etc.

The electrodes are constructed by electrode plates each of which comprises a base of Ti (titan) and a coated layer of Ir (iridium), Pt (platinum).

By making current flow into tap water or the like through the electrodes, hydrogen ion (H⁺) and hydroxide ion (OH⁻) in the water react with each other according to the following reaction (1) at the cathode:

4H⁺ + 4e⁻ + (40H⁻) → 2H₂ + (40H⁻) (1)

Furthermore, water is electrolyzed according to the following reaction formula (2) at the anode:

2H₂O → 4H⁺ + O₂ + 4e⁻ (2)

At the same time, chlorine ion (Cl⁻) contained water (contained in water such as tap water or the like in advance) reacts according to the following reaction formula (3), and chlorine (Cl₂) is generated:

2Cl⁻ → Cl₂ + 2e⁻ (3)

Furthermore, Cl₂ thus generated reacts with water and hypochlorous acid (HClO) and hydrogen chloride (HCl) are generated:

Cl₂ + H₂O → HClO + HCl (4)

In this construction, by supplying current between the electrodes, active oxygen species such as hypochlorous acid having strong sterilizing force, etc. is generated. For example, by supplying the electrolytic water containing hypochlorous acid to the gas-liquid contact member 3, breeding of various bacteria, etc. in the gas-liquid contact member 31 can be prevented, and also air which is passed through the gas-liquid contact member 31 is brought into contact with the electrolytic water, whereby virus, etc. floating in the air passing through the gas-liquid contact member 31 are inactivated (decomposed, etc.) by the electrolytic water.

Furthermore, gaseous materials which cause odor, etc. in the air can be dissolved in the electrolytic water or react with the active oxygen species such as hypochlorous acid, etc. contained in the electrolytic water while passing through the gas-liquid contact member 31, whereby these materials are removed from the air and thus the air can be deodorized by the gas-liquid contact member 31.

When tap water is electrolyzed to generate electrolytic water, scale is liable to occur due to the water quality such as the hardness of tap water or the like.

This embodiment is equipped with a water circulating passage for circulating electrolytic water pumped by the circulating pump 42 through the gas-liquid contact member 31 and the water receiving tray 44 in this order, and an ion exchange resin pack 100 for adjusting the hardness of water is disposed in the water supply tank 41 located out of the water circulating tank 41 as shown in Fig. 2. The ion exchange resin pack 100 is constructed by packing ion exchange resin in a predetermined pack material, and the ion exchange resin can be regenerated by a predetermined regenerating treatment. In this construction, the ion exchange resin pack 100 for softening water such as tap water or the like is provided in the water supply tank 43 located out of the circulating passage of the electrolytic water, so that the circulating electrolytic water in the water circulating passage does not come into contact with the ion exchange resin pack 100. That is, the ion exchange resin pack 100 is used only to soften the water in the water supply tank 41, and it does not come into contact with the circulating electrolytic water. The circulating electrolytic water has been already softened, and it is not required to be brought into contact with the ion exchange resin. In this construction, an interval time required to generate the ion exchange resin can be set to a longer value as compared with a case where the circulating electrolytic water is brought into contact with the ion exchange resin. Furthermore, the ion exchange resin pack 100 is constructed by packing a predetermined amount of ion exchange resin, and water in the water supply tank 41 can be simply softened by merely changing the number of ion exchange resin packs 100 to be input into the water supply tank 41 in accordance with the hardness of water.

Fig. 5 shows an example of the number of ion exchange resin packs 100 to be input into the water supply tank 41. For example, when the hardness of tap water is equal to 100ppm or less, it is not required to input any ion exchange resin pack 100. In the case of the hardness of 100ppm to 200ppm, one ion exchange resin pack 100 may be input, in the case of the hardness of 200ppm to 300ppm, two ion exchange resin packs may be input, and in the case of the hardness of 300ppm to 400ppm, three ion exchange resin packs may be input. The exchange timing of the ion exchange resin pack 100 is reported by turn-on of a filter lamp.

In this construction, tap water is softened by disposing the ion exchange resin pack 100, and thus occurrence of scale can be suppressed. Accordingly, even when the air filtering apparatus 1 is set in a country or district in which the hardness of tap water is high, the electrodes for generating electrolytic water, pipes for supplying electrolytic water to the gas-liquid contact member 31, etc. can be suppressed from being clogged with scale, so that the maintenance time of water exchange can be set to a long value of 60 hours or more, for example.

Furthermore, when electrolytic water is generated, the concentration of active oxygen species in the electrolytic water is adjusted to such a value that virus, etc. to be filtered is inactivated. The adjustment of the concentration of the active oxygen species can be performed by adjusting the voltage to be applied between the electrodes to adjust the value of current flowing between the electrodes.

For example, when current having a predetermined current density (for example, 20mA/cm² or the like) is supplied between the electrodes, electrolytic water containing active oxygen species of a predetermined concentration (for example, free residual chlorine concentration of 1mg/l or the like) can be generated by electrolysis of water. By changing the voltage applied between the electrodes to change the current value, the concentration contained in the electrolytic water can be adjusted. Specifically, by increasing the current value, the concentration of the active oxygen species in electrolytic water can be basically increased.

In the electrolytic bath 43, the voltage value applied between the electrodes, the current value flowing between the electrodes, the current supply time, etc. are preset in accordance with the standard value of the concentration of predetermined ion species such as chloride or the like in tap water at the shipping time. However, the ion species concentration (water quality) such as the chloride ion concentration or the like in tap water is different in accordance with the district (for example, among countries, among districts or the like) in which the air filtering apparatus 1 is set. Therefore, even when power is supplied between the electrodes with the same current value for the same current time, electrolytic water containing a predetermined concentration of active oxygen species cannot be generated in a district where the concentration of the ion species is low, and thus it may be impossible to obtain a desired air filtering effect.

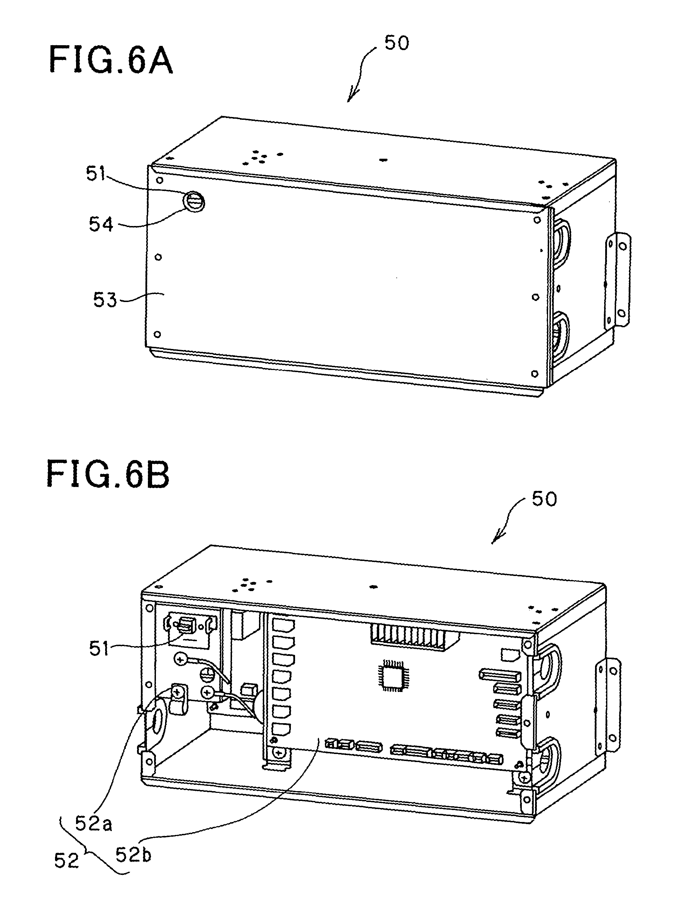

In this embodiment, as shown in Figs. 6A and 6B, a rotational operating tab 51 (operating unit) is provided to the controller 52 (control board 52a) in the electrical component box 50 to change the current conduction state between the electrodes in the electrolytic bath 43 when or after the air filtering apparatus 1 is set (see Fig. 6B) . The electrical component box 50 is used to accommodate various kinds of electrical parts as described above in addition to the control boards 52a and 52b on which CPU, RAM, ROM, etc. as constituent elements of the controller 52 are disposed.

As shown in Fig. 6B, the rotational operating tab 51 is provided to the control board 52a so as to be located at the upper left side of the front face of the electrical component box 50. Furthermore, as shown in Fig. 6A, the rotational operating tab 51 is disposed so as to be exposed to the outside through a hole 54 formed in the front panel 53 of the electrical component box 50.

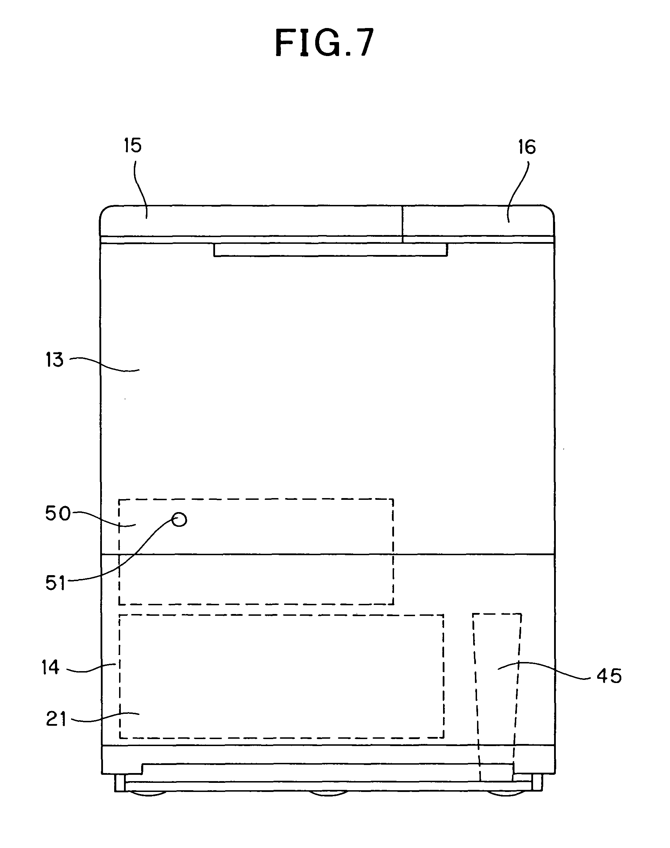

As shown in Fig. 2, the electrical component box 50 is disposed on the upper surface of the partition plate 17, and the rotational operating tab 51 is exposed to the outside when the upper front panel 13 is detached from the housing 10 as shown in Fig. 7. Furthermore, as shown in Fig. 7, the boundary position between the upper front panel 13 and the lower front panel 14 is located substantially at the center position in the vertical direction of the electrical component box 50, and only the upper portion of the electrical component box 50 is exposed to the outside when the upper front panel 13 is detached from the housing 10. In Fig. 7, the electrical component box 50, the air blower 21 and the drain tank 45 are illustrated by broken lines.

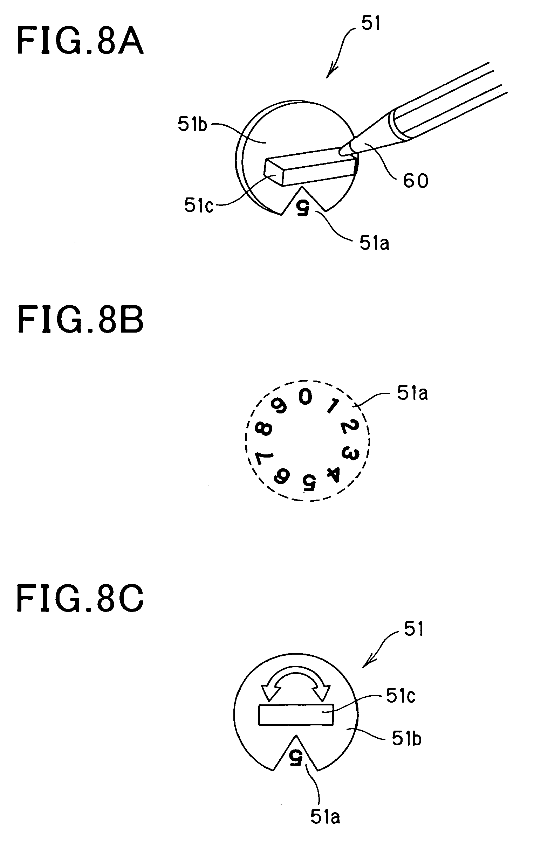

As shown in Figs. 8A to 8C, the rotational operating tab 51 has a disc-shaped electrolysis condition indicating number portion 51a on which electrolysis condition indicating numbers (current supply state indicating numbers) of "0" to "9" are drawn at a predetermined angular interval, and a rotational operating portion 51b which exposes only one of the electrolysis condition indicating numbers drawn on the electrolysis condition indicating number portion 51a while the other numbers are hidden, and has a tab 51c projecting therefrom to the outside.

The rotational operating portion 51b may be provided so as to project from the front panel 53 of the electrical component box 50 to the outside of the electrical component box 50, or may be provided so as to be contained in the hole 54 formed in the front panel 53 of the electrical component box 50. In this embodiment, the rotational operating tab 51 is provided so as to be contained in the hole 54, and thus the tab 51c is rotated by a pen tip 60 or the like as shown in Fig. 8A.

In this embodiment, by rotating the rotational operating portion 51b every predetermined angle, any one of the electrolysis condition indicating numbers (the current supply state indicating numbers) of "0" to "9" can be selected. The controller 52 controls the current supply state so that current of a predetermined current value flows between the electrodes for a predetermined current supply time so as to establish the current supply state associated with the electrolysis condition indicating number selected by the rotational operating tab 51.

Specifically, each electrolysis condition indicating number is associated with a predetermined current supply state, and as the electrolysis condition indicating number increases, the power amount supplied to the electrodes is stepwise increased. At the shipping time, the electrolysis condition indicating number is set to an intermediate number "5", for example, and the electrolysis condition indicating number is changed in accordance with the concentration of ion species of water to be supplied for electrolysis, whereby the current supply state between the electrodes is changed.

According to the this embodiment, water quality adjustment can be easily performed in accordance with the hardness of tap water and the chlorine ion concentration so that scale hardly occur or electrolytic water containing a predetermined concentration of active oxygen species is liable to be generated as shown in Fig. 5. When the hardness of tap water is high, the number of ion exchange resin packs 100 to be input may be changed as described above. The electrolysis condition can be adjusted in accordance with the chlorine ion concentration after the hardness of tap water is adjusted by inputting a desired number of ion exchange resin packs 100. By setting the electrolysis condition indicating number to a predetermined number, electrolysis water containing a desired concentration of active oxygen species is easily generated. In Fig. 5, the electrolysis condition indicating numbers "1" to "7" correspond to "No. 1" to "No. 7" or the like.

In order to increase/reduce the power amount supplied to the electrodes, the current value flowing between the electrodes may be increased/reduced or the current supply time to supply current between the electrodes may be increased/reduced.

Furthermore, some of the electrode condition indicating numbers of "0" to "9" may be associated with the electrode supply power amount which is stepwise varied in accordance with the electrode condition indicating number as described above while the other electrode condition indicating numbers are associated with predetermined current states in a special operation mode, for example. As such a special mode may be considered a scale removing operation mode in which current is supplied between the electrodes while inverting the polarities of the electrodes to remove scale deposited on the electrodes, etc., or a cleaning mode in which electrolysis water containing a higher concentration of active oxygen species than usual and supplied to the gas-liquid contact member 31, etc., thereby cleaning the gas-liquid contact member 31, etc.

According to this embodiment as described above, an air flowing passage from the air suction ports 11 to the air blow-out port 12 is formed in the housing 10 by the air blower 21, and air is brought into contact with electrolytic water in the gas-liquid contact member 31 disposed on the air flowing passage, whereby virus, etc. contained in sucked indoor air are filtered. When the hardness of tap water is high in some country or district where the air filtering apparatus 1 is set, the number of ion exchange resin packs 100 is changed as described above, whereby tap water can be properly softened.

The electrolytic bath 43 has at least a pair of electrodes, and electrolyzes predetermined water to generate electrolytic water. The current supply state between the electrodes in the electrolytic bath 43 is controlled by the controller 52. At this time, the controller 52 is provided with the rotational operating tab 51 for changing the current supply state, and the rotational operating tab 51 is disposed so as to be exposed to the outside of the electrical component box 50 through the hole formed in the electrical component box 50 in which the controller 52 is mounted. Therefore, the current supply state under the electrolysis can be simply changed without opening the electrical component box 50. Furthermore, the electrical component box 50 is disposed in the housing 10, and thus the current state between the electrodes can be prevented from being carelessly changed by a user or the like.

Furthermore, by rotating the rotational operating tab 51, the user or the like can easily stepwise change the current state to one of preset current supply states of plural stages.

The electrical component box 50 is disposed in the housing 10 so that the rotating operating tab 51 is exposed to the outside when the upper front panel 13 is detached from the housing 10. Therefore, the rotational operating tab 51 can be easily rotated to change the current supply state between the electrodes. Furthermore, the electrical component box 50 is mounted inside the upper front panel 13, whereby the rotational operating tab 51 can be prevented from being exposed to the outside of the housing 10, so that's the current supply state between the electrodes can be prevented form being carelessly changed by the user or the like. Furthermore, the rotational operating tab 51 is contained in the hole formed in the front panel 53 of the electrical component box 50, so that careless operation by the user or the like can be further prevented.

The above embodiment is one embodiment of the present invention, and thus various modifications may be properly made without departing from the subject matter of the present invention.

For example, in the above embodiment, the air filtering apparatus 1 is equipped with the on-floor mount type housing 10, however, the mount style of the air filtering apparatus 1 is not limited to the on-floor mount type. For example, it may be a wall-suspended type, a ceiling-suspended type or an in-ceiling embedded (cassette) type, that is, the mount style is not limited to a special one.

Furthermore, the electrical component box 50 is disposed in the housing so that the rotational operating tab 51 is exposed to the outside when the upper front panel 13 is detached from the housing 10. However, the electrical component box 50 may be disposed so that the rotational operating tab 51 is exposed to the outside when the lower front panel 14 is detached. In short, the locating position of the electrical component box 50 in the housing 10 is not limited insofar as the electrical component box 50 is disposed inside the freely detachable or freely openable/closable external packaging panel so that the rotational operating tab 51 is exposed to the outside when the outer packaging panel is opened or detached.

Still furthermore, the electrical component box is provided with a peaked portion 55 projecting to the outside of the electrical component box, and the operating unit is provided below the peaked portion 55 as shown in Fig. 2.

## Claims

1. An air filtering apparatus that is equipped with an air blower (21) and a gas-liquid contact member (31) in a housing (10) and in which electrolytic water is brought into contact with indoor air sucked by the air blower to filter the air, and then the filtered air is blown out to an indoor space, **characterized by** further comprising:
a water supply tank (41)for stocking water;
a water receiving tray (44) for temporarily stocking water flowing out from the water supply tank (41);
a circulating pump (42) for pumping up water stocked in the water receiving tray;
an electrolytic water generator (43) for generating electrolytic water from the water pumped by the circulating pump (42); and
an electrolytic water circulating passage for circulating electrolytic water through the gas-liquid contact member and the water receiving tray in this order, wherein at least one ion exchange resin pack (100) for adjusting the hardness of water is provided in the water supply tank is located out of the circulating passage of the electrolytic water.

2. The air filtering apparatus according to claim 1, wherein the electrolytic water generator (43) is equipped with at least one pair of electrodes and a controller for controlling a current supply state between the electrodes, and the controller is provided with an operating unit (51) for changing the current supply state between the electrodes.

3. The air filtering apparatus according to claim 2, further comprising an electrical component box (50) in which the controller is mounted, and the electrical component box is provided with a hole (54) through which the operating unit (51) is exposed to the outside of the electrical component box.

4. The air filtering apparatus according to claim 3, wherein the operating unit stepwise changes the current supply state between the electrodes to one of preset current supply stages of plural stages by rotating the operating unit.

5. The air filtering apparatus according to any one of claims 2 to 4, wherein the housing is equipped with a freely-detachable outer packaging panel (13), and the electrical component box is disposed in the housing so that the operating unit is exposed to the outside when the outer packaging panel (13) is detached.

6. The air filtering apparatus according to any one of claims 2 to 5, wherein the electrical component box is provided with a peaked portion (55) projecting to the outside of the electrical component box, and the operating unit is provided below the peaked portion (55).

7. The air filtering apparatus according to any one of claims 2 to 6, wherein the operating unit changes a power amount to be supplied to the electrodes.

8. The air filtering apparatus according to any one of claims 2 to 7, wherein the operating unit changes a current supply time to supply current between the electrodes.

9. The air filtering apparatus according to any one of claims 2 to 8, wherein the operating unit changes a current value flowing between the electrodes.
